# EUROPEAN PATENT SPECIFICATION

(11) **EP 2 022 377 B1**
(45) Date of publication and mention of the grant of the patent: **16.01.2013**
(21) Application number: 08013490.1
(22) Date of filing: 26.07.2008
(51) Int. Cl.: A47F 9/00

(54) **Display counter unit for newsstands, tobacco shops, stationery shops, and the like**
Verkaufstheke für Zeitungskioske, Tabakwarenläden, Schreibwarenläden u.ä.
Unité de module de comptoir pour kiosques à journaux, débits de tabac, papeteries et similaires

(30) Priority: 06.08.2007 IT TO20070106
(43) Date of publication of application: 11.02.2009
(73) Proprietor: Mezzi Alternativi, 10010 S. Martino Canavese (IT)
(72) Inventor: Casati, Giuseppe, 10010 S. Martino Canavese (IT)
(74) Representative: Spandonari, Carlo

(56) References cited:
- EP-A- 0 575 275
- DE-U1- 8 513 468
- US-A1- 2002 046 508
- US-B1- 6 561 317

## Description

The present invention relates to an display counter unit for shops such as newsstands, tobacco shops, stationery shops, and the like.

As known, counters are installed in shops such as the above-mentioned ones for separating the area occupied by the dealer from the area occupied by the customers. A counter consists of a plurality of counter units which are arranged side-by-side and are bolted to one another, with a top applied on top of them. A conventional counter unit is made of wood or other wood-based material having properties similar to wood, and is typically box-shaped with a face facing the area occupied by the dealer, which is open and is provided with shelves and/or drawers in which the dealer can arrange goods, and a closed, opposite face facing the area occupied by the customers.

EP 575 275 A1 describes a counter unit entirely made of cardboard and comprising a tubular body which is bent to form a peripheral wall with two opposite, closed side walls, a closed rear well and a closed front wall. The counter unit is also closed at its upper end and its lower end by respective plates having stiffening peripheral edges adapted to restreinedly cooperate with respective connection teeth projecting from the upper edges and the lower edges of the tubular body. An advertising panel mounted on posts is installed on the counter unit, with two uprights of the panel which are inserted into respective openings formed on the upper plate. A similar counter unit made of cardboard is described in DE 8 513 468 Ul.

It is known, e.g., from EP 1 114 600 of Applicant, to bolt a portal to the counter unit, on which magazine stands, dispensers of packets of cigarettes, and the like, can be hanged.

It is a main object of the present invention to provide an display counter unit with portal, which allows an easier and more flexible installation of the portal with respect to the conventional counter units, with possibility of installing the portal either on a single counter unit or on a counter consisting of a plurality of units arranged side-by-side.

It is a further object of the invention to provide an display counter unit having relatively low manufacturing costs.

The above objects and other advantages, which will better appear from the following description, are achieved by the display counter unit having the features recited in claim 1, while the dependent claims state other advantageous, though secondary features of the invention.

The invention will be now described in more detail, with reference to a few preferred, non-exclusive embodiments, shown by way of non-limiting example in the attached drawings, wherein:
Fig. 1 is a broken-away, perspective view of a counter unit according to the invention in which a portal is installable;
Fig. 2 is a diagrammatical view in horizontal, cross-section of the peripheral wall of the counter unit according to the invention;
Fig. 3 is a perspective view showing a first, isolated part of the counter unit of Fig. 1;
Fig. 4 is a broken-away, perspective view of an upper portion of the counter unit of Fig.1, in which some parts have been removed for better clarity of illustration;
Fig. 5 is a perspective view showing a second, isolated part of the counter unit of Fig. 1;
Fig. 6 is a broken-away, front view to an enlarged scale of a detail of the counter unit of Fig. 1;
Fig. 7 is a perspective, rear view of a lower portion of the counter unit of Fig. 1;
Fig. 8 is view in horizontal cross-section made along plane VIII-VIII of a detail of the counter unit of Fig. 1;
Fig. 9 is a broken-away, perspective view of a counter unit with a portal according to the invention installed thereon;
Fig. 10 is a perspective view of a counter unit with a portal according to the invention installed thereon;
Fig. 11 shows a first alternative embodiment of the invention;
Fig. 12 shows a second alternative embodiment of the invention;
Fig. 13 shows a third alternative embodiment of the invention;
Fig. 14 shows a fourth alternative embodiment of the invention.

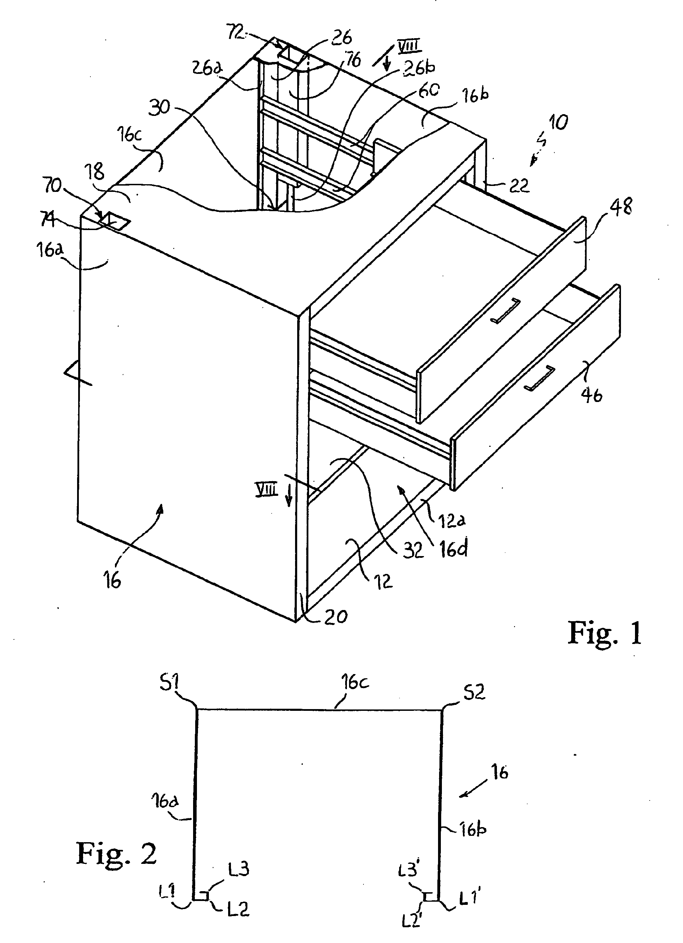

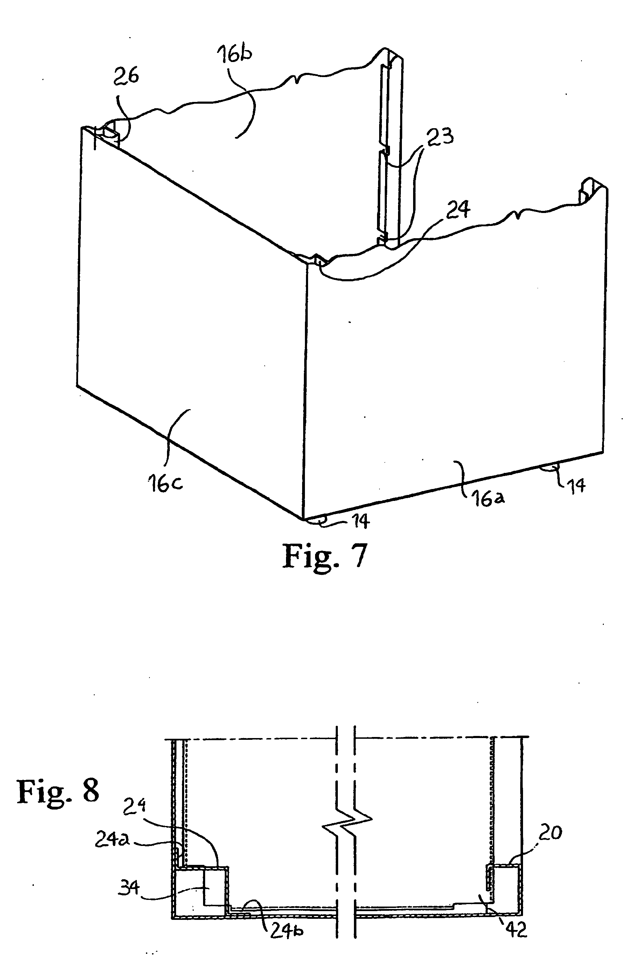

With particular reference to Fig. 1, a counter unit 10 has a rectengular bottom base 12 made of a steel sheet and supported on four adjustable feet such as 14 (Fig. 7). The bottom base is surrounded by a peripheral wall 16 made of a steel sheet, which consists of two closed, opposite side walls 16a, 16b, a closed rear wall 16c facing the area of the shop which is occupied by the customers, and an open front face 16d facing the area occupied by the dealer, through which the dealer may access to the inside of the unit. Bottom base 12 has an edge 12b projecting at right angles downwards at the open side of the counter. The counter unit is closed at its upper end by a rectangular cover 18 made of a steel sheet, which also has an edge 18a projecting at right angles downwards at the open side of the counter.

Having now particular reference to Fig. 2, peripheral wall 16 of the counter unit consists of a steel sheet which is bent at right angles along two vertical lines defining the rear corners S1, S2 of the counter unit. The lateral edges of the steel sheet are each bent at right angles three times along respective three subsequent vertical lines L1, L2, L3 and L1', L2', L3', thereby defining two hollow uprights 20, 22 which laterally bound the open, front face 16d of the counter unit. A plurality of rectangular cuts 23 (see also Fig. 8) are formed along uprights 20, 22, which mainly engage the innermost side of the upright, i.e., the last portion of the edge of the steel sheet between line L3, L3' and the border.

The rear corners of the counter unit each have a steel strip internally welded thereto, which is axially bent at right angles in the opposite direction with respect to the corresponding corner (see also Figs. 7, 8), thereby defining a column 24, 26. The opposite longitudinal edges 24a, 24b and 26a, 26b of the strip are bent at right angles outwards and are welded to the rear wall 16c and to the adjacent, side wall 16a, 16b of the counter unit respectively. A plurality of horizontal slots such as 30 are formed along the columns. The slots are aligned to the cuts 23 in the uprights and partially cut both the sides of the column.

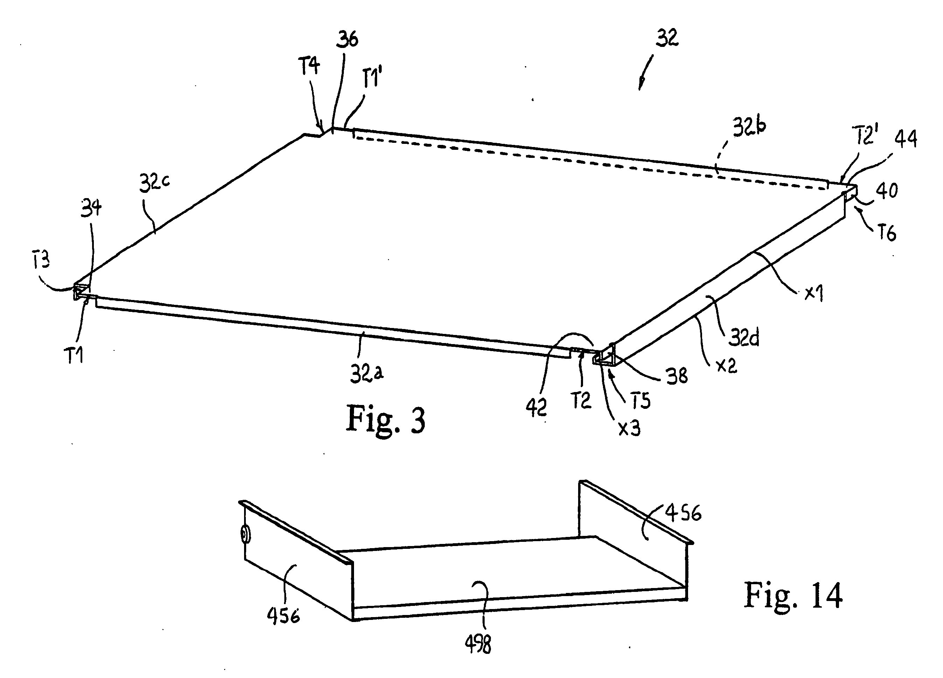

A shelf 32 (separately shown in Fig. 3) is removably supported within the counter unit. The shelf mainly consists of a substantially rectangular steel plate which has two lateral, stiffening ribs 32a, 32b which project downwards at right angles from its opposite, lateral edges and extend along the whole length of the edge except for short spans T1, T2 and T1', T2' at the opposite ends of the edge. A rear projection 32c projects from the rear end of the plate, extends along the whole length of the rear edge except for short spans T3, T4 at the ends of the edge, and has an end portion bent at right angles downwards for stiffening purposes. Accordingly, two flat tabs 34, 36 are defined between the rear projection 32c and the two lateral, stiffening ribs 32a, 32b, which are insertable into the horizontal slots 30 in columns 24, 26 to support the shelf at its rear end. A front projection 32d projects from the front end of the plate and extends along the whole length of the edge except for short spans T5, T6 at the ends of the edge. Front projection 32d is first bent at right angles downwards along a first line X1, and then is further bent at right angles inwards along two subsequent horizontal lines X2, X3. Two teeth 38, 40 project downstream from the front end of the plate near the opposite ends of front projection 32d. Accordingly, two toothed tabs 42, 44 are defined between front projection 32d and the two lateral, stiffening ribs 32a, 32b, which are restrainedly engageable into cuts 23 to support the shelf at its front end.

In order to remove the shelf, the front end of the shelf is lifted so that front toothed tabs 42, 44 are disengaged from cuts 23, then the shelf is pulled away towards the open, front face 16d of the unit so that rear, flat tabs 34, 36 are disengaged from slots 30. To this purpose, the height of slots 30 preferably is such that the rear tabs are received in the slots with a loose fit.

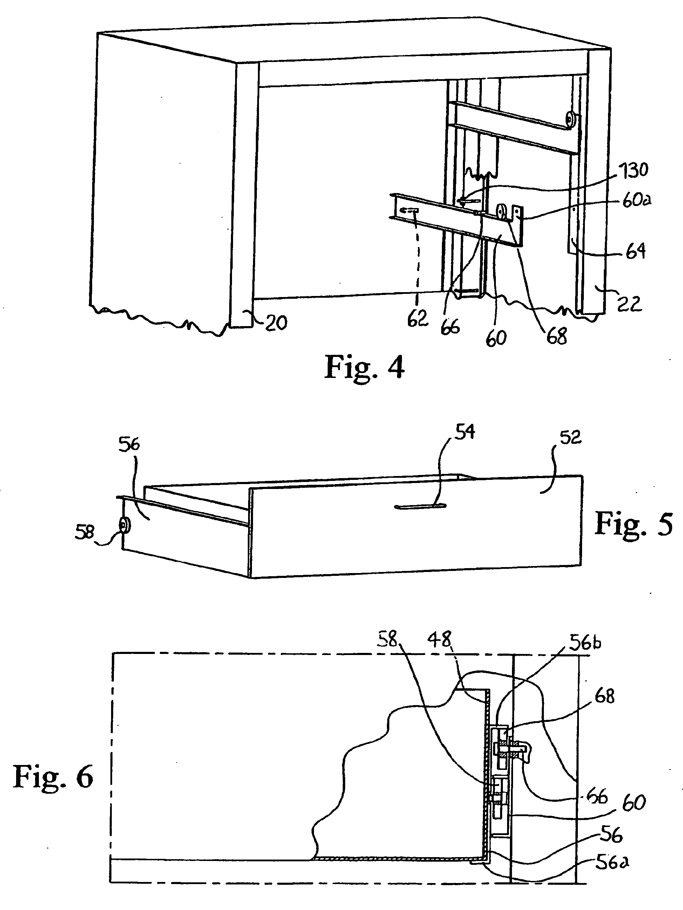

Having now particular reference to Figs 4-6, two drawers 46, 48 having a rectangular profile are supported within counter unit 10, each of which is provided with a front panel 52 (Fig. 5) with handle 54. Each of the opposite, lateral sides of the drawer has a guide welded thereto, which consists of a steel plate 56 welded to the lateral side of the drawer and having a lower edge 56a bent at right angles under the bottom of the drawer, and an upper, cantilever edge 56b bent at right angles outwards (Fig. 6). Each of the guides has a roller 58 pivoted to its rear end. The drawer is supported within the counter unit on a pair of tracks each consisting of a channel 60 arranged with the parallel sides of the C-shape projecting inwards. Channel 60 has a pin 62 welded near its rear end, which is received in a recess 63 formed in the corresponding column 24, 26 for supporting the channel at its rear end. Channel 60 has a flange 60a projecting upwards from its front end. The flange is fixed to a box 64, which is welded to the rear side of the corresponding upright 20, 22, by a screw 66 about which another roller 68 is pivoted. When the drawer is inserted in the counter unit, upper edge 56b of guide 56 lies on roller 68, which is hinged to the counter, while roller 58, which is hinged to the drawer, is slidably received between the parallel sides of channel 60, thereby preventing the drawer from tilting. Preferably, the drawer is sized in such a way that a gap is defined between the rear wall of the drawer and the inner rear wall of the counter, for receiving wires supplying any accessory equipment associated to the counter unit.

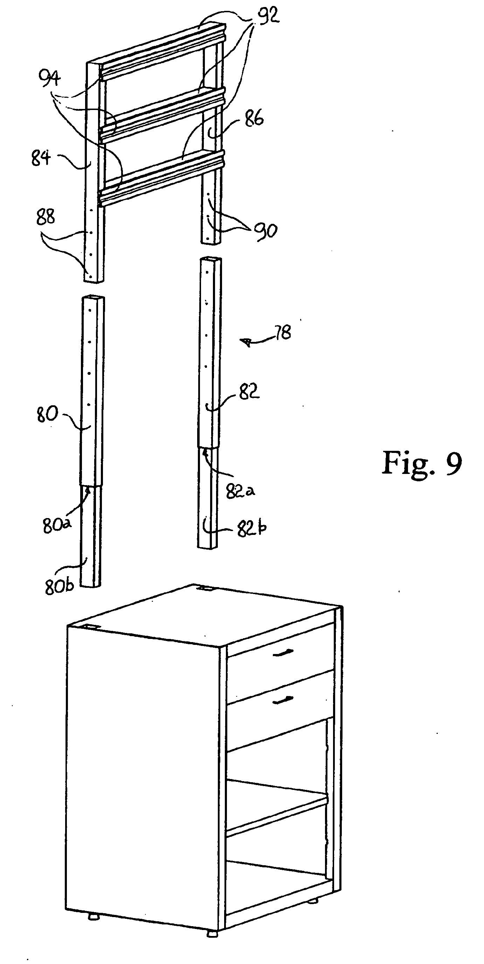

Having now particular reference to Figs. 1, 9 and 10, the counter unit according to the invention allows a portal 70 to be easily and flexibly installed/removed. The portal is adapted to support dispensers of packets of cigrettes, such as those shown in Patent document EP 1 114 600, as well as magazine stands, and the like. To this purpose, cover 18 has two rectangular openings 70, 72 near respective rear corners S1, S2 of the unit, which are aligned along a direction parallel to rear wall 16c of unit 10. A pair of vertical sleeves 74, 76 project from openings 70, 72 to the inside of the unit. More particularly, sleeves 74, 76 each consist of a box arranged adjacent to a respective side wall 16a, 16b and to the rear column 24, 26 on the same side, in the gap defined between the side wall and the track 60 supporting the drawer.

As mentioned above, a portal 78 is installable on the counter unit. The portal comprises two tubular uprights 80, 82 having a rectangular profile, whose lower ends 80a, 82a have thin feet 80b, 82b axially projecting therefrom, which are slidably insertable into sleeves 74, 76 respectively. Rods 84, 86 are received within the tubular uprights and are lockable at a desired heigth by screws (not shown) inserted in holes such as 88, 90. Rods 84, 86 are interconnected by crosspieces 92 to form a rigid frame. Crosspieces 92 carries a respective hat 94 having two skirts which, together with crosspiece 92, define upward and downward slots respectively.

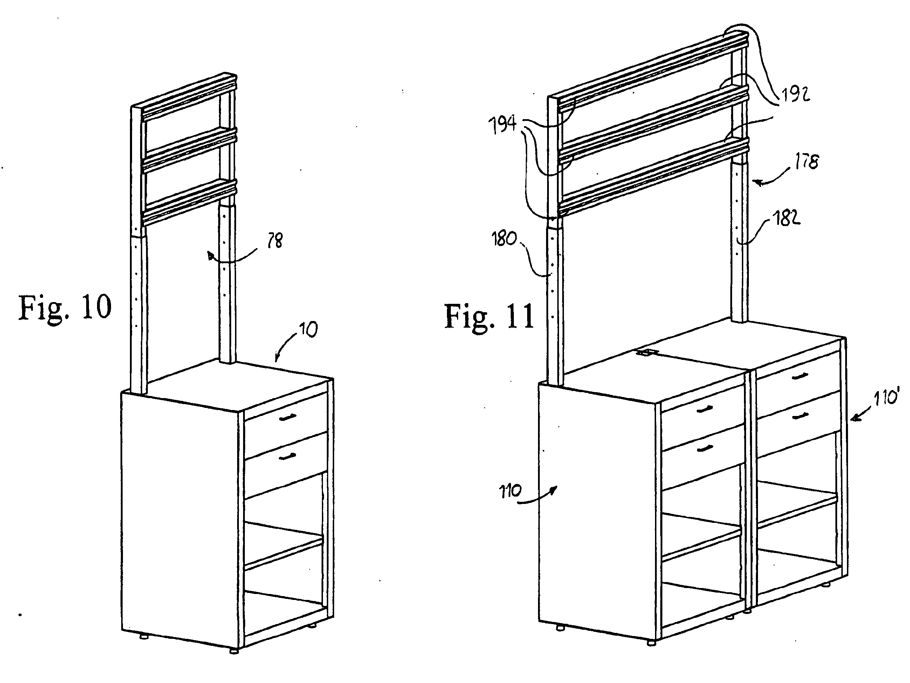

In order to install portal 78 on the unit, narrow feet 80b, 82b of uprights 80, 82 are inserted into sleeves 74, 76, with the lower ends 80a, 82a of the uprights laying on cover 18 (Fig. 10).

Portal 78 is adapted to support suspended units of a known type (not shown) such as the above mentioned ones, which are provided with hooks each consisting of shaped steel plate insertable into the slots defined by the skirts, similarly to what described in the above-cited EP 1114 600 with reference to dispensers of packets of cigarettes.

An alternative embodiment of the invention in shown in Fig. 11, wherein two units 110,110' arranged side-by-side, with their side walls interconnected by bolts, support a portal 178 of the above mentioned type, which is provided with elongated crosspieces 192 and with correspondingly elongated hats 194, whereby the two tubular uprights 180,182 have their feet inserted into the outermost sleeves of the two counter units.

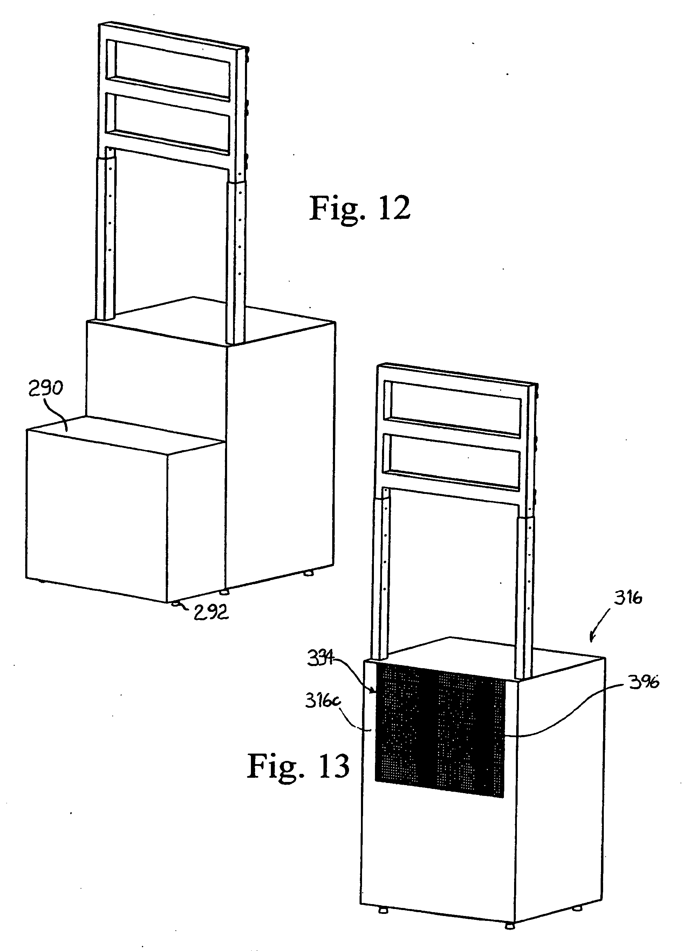

Another alternative embodiment of the invention in shown in Fig. 12, wherein a box 290 made of a metal sheet is bolted to rear wall 216c of counter unit 210, is supported on feet such as 292, and may support stands and/or general goods to be exhibited.

A further alternative embodiment of the invention in shown in Fig. 13, wherein rear wall 316c of counter unit 310 has a rectangular opening 394 provided with a wire net 396 which can be used for hanging goods-holding/magazine-holding brackets, and the like.

Fig. 14 shows another alternative embodiment of the invention, wherein the drawer is replaced by a wooden slidable shelf 498, e.g., for a computer keyboard. Slidable shelf 498 has a pair of guides 456 attached to its opposite sides, which are equal to the guides of the drawers and are slidable along the same tracks, so that no further description will be given about them.

A few preferred embodiments of the invention have been described herein, but of course many changes may be made by a person skilled in the art within the scope of the claims. In particular, with changes which will be apparent to the person skilled in the art, the above described system for supporting the portal can be associated to any other counter units in which, e.g., the shelves are welded or bolted to the side walls rather than supported by slots and cuts formed inside the unit near the corners. Of course, the counter unit, or the set of counter units forming a counter, can be conventionally lined, e.g., with wooden panels and with a top which should be bored at the openings on the cover for receiving the uprights of the portal. Nonetheless, when it is known from the beginning that an enlarged portal 170 of the type described in Fig. 11 will be installed on a pair of units arranged side-by-side, each of the units may have a sole opening with a respective sleeve near one of its corners.

## Claims

1. An display counter unit (10) for shops, comprising
- a bottom base (12),
- a peripheral wall (16) rising form the periphery of the bottom base (12) and consisting of two opposite, closed side walls (16a, 16b), and a rear wall (16c) and an open front face (26d),
- a cover (18) closing the upper end of said peripheral wall (16), and having at least one opening (70, 72),
**characterized in that** said at least one opening (70, 72) has a vertical sleeve (74, 76) projecting therefrom to the inside of the unit for receiving a respective supporting projection (80b, 82b) of a portal (78).

2. The display counter unit of claim 1, **characterized in that** said at least one opening is positioned near a respective rear corner (S1, S2) of the unit.

3. The display counter unit of claim 2, in which two columns (24, 26) are attached to the inside of the unit between the rear wall (16c) and two respective side walls (16a, 16b), **characterized in that** said sleeve (74, 76) is arranged adjacent to a respective side wall (16a, 16b) of the unit and to the corresponding column (24, 26).

4. The display counter unit of any of claims 1 to 3, **characterized in that** it comprises two of said openings (70, 72) with respective vertical sleeves (74, 76), which are positioned near respective two rear corners (S1, S2) of the unit.

5. The display counter unit of claim 1, **characterized in that** it comprises a portal (78) having a pair of feet (80b, 82b) each slidably inserted into a respective one of said openings (70, 72).

6. The display counter unit of any of claims 1 to 5, **characterized in that** the rear wall (316c) of the unit has a rectangular opening (394) provided with a wire net (396).

7. The display counter unit of any of claims 1 to 6, **characterized in that** a box (290) is anchored to the rear side of the unit, which is lower than the unit.

8. The display counter unit (10) of claim 5, **characterized in that** said portal (78, 178) is provided with at least two uprights (80, 82) interconnected by crosspieces (92), each of said uprights being provided of a respective one of said feet (80b, 82b) which is slidably insertable into a respective sleeve (74, 76) of the counter unit through a respective one of said openings.

9. A counter for shops, **characterized in that** it comprises a pair of counts units (110, 110') according to claim 1 which are arranged side-by-side, and a portal (178) having a pair of feet each inserted into a respective one of the openings of said counter units.

## Patentansprüche

1. Verkaufstheke (10) für Geschäfte, mit
- einem Boden (12),
- einer sich von dem Rand des Bodens (12) nach oben erstreckenden Umfangswand (16), die aus zwei gegenüberliegenden, geschlossenen Seitenwänden (16a, 16b), einer Rückwand (16c) und einer offenen Vorderfläche (16d) besteht,
- einer Abdeckung (18), die das obere Ende der Umfangswand (16) verschließt und zumindest eine Öffnung (70, 72) aufweist,
**dadurch gekennzeichnet, dass** die zumindest eine Öffnung (70, 72) eine sich aus ihr zur Innenseite der Theke ragende, vertikale Hülse (74, 76) zur Aufnahme eines entsprechenden Haltevorsprungs (80b, 82b) eines Portalrahmens (78) umfasst.

2. Verkaufstheke nach Anspruch 1,
**dadurch gekennzeichnet, dass** die zumindest eine Öffnung nahe einer entsprechenden hinteren Ecke (S1, S2) der Theke angeordnet ist.

3. Verkaufstheke nach Anspruch 2, bei der zwei Säulen (24, 26) zwischen der Rückwand (16c) und zwei zugehörigen Seitenwänden (16a, 16b) an der Innenseite der Theke angebracht sind,
**dadurch gekennzeichnet, dass** die Hülse (74, 76) an eine zugehörige Seitenwand (16a, 16b) der Theke und die entsprechende Säule (24, 26) angrenzend angeordnet ist.

4. Verkaufstheke nach einem der Ansprüche 1 bis 3,
**dadurch gekennzeichnet, dass** sie zwei der Öffnungen (70, 72) mit zugehörigen vertikalen Hülsen (74, 76) aufweist, die nahe zwei entsprechenden, hinteren Ecken (S1, S2) der Theke angeordnet sind.

5. Verkaufstheke nach Anspruch 1,
**dadurch gekennzeichnet, dass** sie einen Portalrahmen (78) mit einem Paar Füße (80b, 82b) aufweist, die je verschieblich in eine entsprechende der Öffnungen (70, 72) eingeführt sind.

6. Verkaufstheke nach einem der Ansprüche 1 bis 5,
**dadurch gekennzeichnet, dass** die Rückwand (316c) der Theke eine rechteckige Öffnung (394) umfasst, die mit einem Drahtnetz (396) versehen ist.

7. Verkaufstheke nach einem der Ansprüche 1 bis 6,
**dadurch gekennzeichnet, dass** ein Behälter (290) an der Rückseite der Theke befestigt ist, der niedriger ist als die Theke.

8. Verkaufstheke (10) nach Anspruch 5,
**dadurch gekennzeichnet, dass** der Portalrahmen (78, 178) mit wenigstens zwei Pfosten (80, 82) versehen ist, die durch Querstücke (92) miteinander verbunden sind, wobei jeder der Pfosten mit einem zugehörigen der Füße (80b, 82b) versehen ist, der verschieblich in eine zugehörige Hülse (74, 76) der Verkaufstheke durch eine zugehörige der Öffnungen einsteckbar ist.

9. Theke für Geschäfte,
**dadurch gekennzeichnet, dass** sie ein Paar Verkaufstheken (110, 110') nach Anspruch 1 aufweist, die nebeneinander angeordnet sind, und einen Portalrahmen (178) mit einem Paar Füße, die jeweils in eine zugehörige der Öffnungen der Verkaufstheken eingeführt sind.

## Revendications

1. Unité de module de comptoir (10) pour magasins, comprenant :
une base inférieure (12),
une paroi périphérique (16) remontant à partir de la périphérie de la base inférieure (12) et se composant de deux parois latérales fermées opposées (16a, 16b), et d'une paroi arrière (16c) et d'une face frontale ouverte (16d),
un couvercle (18) fermant l'extrémité supérieure de ladite paroi périphérique (16) et ayant au moins une ouverture (70, 72),
**caractérisée en ce que** ladite au moins une ouverture (70, 72) a un manchon vertical (74, 76) faisant saillie de cette dernière jusqu'à l'intérieur de l'unité pour recevoir une saillie de support (80b, 82b) respective d'un portique (78).

2. Unité de module de comptoir selon la revendication 1, **caractérisée en ce que** ladite au moins une ouverture est positionnée à proximité d'un coin arrière (S1, S2) respectif de l'unité.

3. Unité de module de comptoir selon la revendication 2, dans laquelle deux colonnes (24, 26) sont fixées à l'intérieur de l'unité entre la paroi arrière (16c) et les deux parois latérales (16a, 16b) respectives, **caractérisée en ce que** ledit manchon (74, 76) est agencé de manière adjacente à une paroi latérale (16a, 16b) respective de l'unité et à la colonne (24, 26) correspondante.

4. Unité de module de comptoir selon l'une quelconque des revendications 1 à 3, **caractérisée en ce qu'**elle comprend deux desdites ouvertures (70, 72) avec des manchons verticaux (74, 76) respectifs qui sont positionnés à proximité des deux coins arrière (S1, S2) respectifs de l'unité.

5. Unité de module de comptoir selon la revendication 1, **caractérisée en ce qu'**elle comprend un portique (78) ayant une paire de pieds (80b, 82b), chacun inséré de manière coulissante dans une ouverture respective desdites ouvertures (70, 72).

6. Unité de module de comptoir selon l'une quelconque des revendications 1 à 5, **caractérisée en ce que** la paroi arrière (316c) de l'unité a une ouverture rectangulaire (394) prévue avec un treillis (396).

7. Unité de module de comptoir selon l'une quelconque des revendications 1 à 6, **caractérisée en ce qu'**un boîtier (290) est ancré sur le côté arrière de l'unité, qui est plus bas que l'unité.

8. Unité de module de comptoir (10) selon la revendication 5, **caractérisée en ce que** ledit portique (78, 178) est dotée d'au moins deux montants (80, 82) interconnectés par des traverses (92), chacun desdits montants étant prévu avec un pied respectif desdits pieds (80b, 82b) qui peut être inséré de manière coulissante dans un manchon (74, 76) respectif de l'unité de comptoir à travers une ouverture respective desdites ouvertures.

9. Comptoir pour magasins, **caractérisé en ce qu'**il comprend une paire d'unités de comptoir (110, 110') selon la revendication 1, qui sont agencées côte à côte, et un portique (178) ayant une paire de pieds, chacun inséré dans une ouverture respective des ouvertures desdites unités de comptoir.
